# EUROPEAN PATENT APPLICATION

(11) **EP 3 819 376 A1**
(43) Date of publication of application: **12.05.2021**
(21) Application number: 19831347.0
(22) Date of filing: 27.05.2019
(51) Int. Cl.: C12N 15/10, C12N 15/113, C12Q 1/6851, C12Q 1/686, C12Q 1/6876

(54) **COMPOSITION AND METHOD FOR IMPROVING EFFICIENCY OF SMALL RNA EXTRACTION**

(30) Priority: 06.07.2018 JP 2018129064
(71) Applicant: Daiyukai Health System, Ichinomiya-shi, Aichi 491-8551 (JP)
(72) Inventor: KIKUCHI, Arizumi, Ichinomiya-shi, Aichi 491-0113 (JP); SAWAMURA, Takahiro, Ichinomiya-shi, Aichi 491-0113 (JP)
(74) Representative: HGF
(86) International application number: PCT/JP2019/020838
(87) International publication number: WO 2020/008752

(57) **Abstract**

Provided is an agent for improving efficiency of small RNA extraction comprising the following components: (a) polyethylene glycol, and (b) tetraethylene glycol dimethyl ether.

SEQUENCE LISTING

<110>  Daiyukai Health System

<120>  Compositions and methods for enhancing small RNA extraction
       efficacy

<130>  3057-PCT

<150>  JP 2018-129064
<151>  2018-07-06

<160>  4

<170>  PatentIn version 3.5

<210>  1
<211>  22
<212>  RNA
<213>  Homo sapiens

<400>  1
uagcuuauca gacugauguu ga                                              22


<210>  2
<211>  50
<212>  DNA
<213>  Artificial Sequence

<220>
<223>  Stem-loop RT primer

<400>  2
gtcagaggag gtgcagggtc cgaggtattc gcacctcctc tgactcaaca                50


<210>  3
<211>  24
<212>  DNA
<213>  Artificial Sequence

<220>
<223>  Forward primer

<400>  3
gcctgctagc ttatcagact gatg                                            24


<210>  4
<211>  16
<212>  DNA
<213>  Artificial Sequence

<220>
<223>  Reverse primer

<400>  4
gtgcagggtc cgaggt                                                     16

## Description

### [Technical Field]

The present invention relates to an agent for enhancing small RNA extraction efficacy and relates to a method for isolating RNA using the same.

### [Background Art]

A microRNA (miRNA) is a small single-stranded non-coding RNA having a length of about 21 to 25 bases; more than 20,000 miRNAs have been identified thus far (http://mirbase.org/). It has been demonstrated that these miRNAs have functions of controlling mRNA expression by binding to target mRNA, and they are involved in various biological processes such as cell proliferation, differentiation, and apoptosis. It is estimated that expression of about 30% of human genes is regulated by miRNA. In recent years, it has been suggested that abnormal expression of miRNA affects the onset and the pathological conditions of various diseases including cancer (Non-Patent Documents 1 and 2). The miRNAs are contained in exosomes secreted from cells and are present in body fluids such as blood and urine. Therefore, minimally invasive liquid biopsies, which diagnose diseases by detecting and quantifying miRNAs in body fluids, have been drawing attention.

Quantitative real-time RT-PCR is generally performed as a simple and rapid method for quantifying miRNA. Real-time RT-PCR is a method suitable for making a diagnosis in a clinical setting since the results can be obtained in a short time of about several hours, and it can be carried out using simple devices. Here, it is necessary to isolate the miRNA with high accuracy and high yield in order to detect and quantify miRNA by real-time RT-PCR. The Boom method, which is based on the principle that nucleic acid molecules are adsorbed on a silica carrier in the presence of a chaotropic salt, is generally used as a method for isolating total RNA (Non-Patent Document 3). However, under the normal conditions of the Boom method, low-molecular-weight RNA such as miRNA can only be captured with low efficiency. Therefore, there was a problem that a sufficient amount of miRNA for real-time RT-PCR analysis could not be obtained. Thus, there is a demand for a method that can extract total RNA including miRNA at a high yield, while being as easy as conventional methods.

### [Citation List]

### [Non-Patent Document]

[Non-Patent Document 1] Ochiya, T., Drug Delivery System, Vol. 26, No. 1, pp. 10-14 (2011)
[Non-Patent Document 2] Rupaimoole, R. et al, Nat. Rev. Drug Discov., Vol. 16, No. 3, pp. 203-222 (2016)
[Non-Patent Document 3] Boom, R. et al., J. Clin. Microbiol., Vol. 28, No. 3, pp. 495-503 (1990)

### [Summary of Invention]

### [Technical Problem]

The object of the present invention is to solve the problems of the prior art and to provide a method for extracting miRNA from a biological sample with high yield and high accuracy by a safe and simple operation.

### [Solution to Problem]

The present inventors have earnestly researched and, as a result, have found that the miRNA extraction efficiency of the Boom method is remarkably improved by adding a high-molecular-weight polyethylene glycol to a nucleic acid extract containing a chaotropic salt. However, high-molecular-weight polyethylene glycols are solid at room temperature, and even if heated and melted, they are extremely viscous and are therefore difficult to handle with a pipette.

Thus, the present inventors have earnestly researched a composition having excellent operability while maintaining the effect of improving the miRNA extraction efficiency of high-molecular-weight polyethylene glycol. As a result, the present inventors have found that a mixture of polyethylene glycol and tetraethylene glycol dimethyl ether has excellent operability and stability, and can improve the miRNA extraction efficiency to the same extent as high-molecular-weight polyethylene glycol.

Specifically, according to one embodiment, the present invention provides an agent for improving efficiency of small RNA extraction comprising the following components: (a) polyethylene glycol, and (b) tetraethylene glycol dimethyl ether.

Preferably, the polyethylene glycol has an average molecular weight of from 600 to 2000.

Preferably, the ratio of the component (a) to the component (b) is from 3:7 to 6:4.

The content of the component (a) is preferably from 20 to 60 vol%, and the content of the component (b) is preferably from 25 to 70 vol%, based on the agent for improving efficiency of small RNA extraction.

Preferably, the agent for improving efficiency of small RNA extraction further contains a chaotropic salt.

The chaotropic salt is preferably guanidine thiocyanate.

Preferably, the agent for improving efficiency of small RNA extraction is in liquid form in the temperature range of 15 to 35°C, and has a viscosity that allows measuring by a micropipette.

According to one embodiment, the present invention also provides a method for extracting small RNA from a sample, the method comprising: (i) a step of providing a sample containing small RNA; (ii) a step of adding a solution containing a chaotropic salt to the sample; (iii) a step of adding the above agent for improving efficiency of small RNA extraction to a first mixture obtained in the step (ii); (iv) a step of contacting a second mixture obtained in the step (iii) with a nucleic acid-binding carrier containing silica, whereby the small RNA binds to the nucleic acid-binding carrier; and (v) a step of eluting the small RNA which is bound to the nucleic acid-binding carrier.

According to one embodiment, the present invention also provides a method for analyzing microRNA from a biological sample, the method comprising: (i) a step of providing a biological sample containing microRNA; (ii) a step of adding a solution containing a chaotropic salt to the biological sample; (iii) a step of adding the above agent for improving efficiency of small RNA extraction to a first mixture obtained in the step (ii); (iv) a step of contacting a second mixture obtained in the step (iii) with a nucleic acid-binding carrier containing silica, whereby total RNA including the microRNA binds to the nucleic acid-binding carrier; (v) a step of eluting the total RNA which is bound to the nucleic acid-binding carrier; and (vi) a step of amplifying the microRNA in the total RNA obtained in the step (v) by quantitative RT-PCR.

The biological sample is preferably a body fluid or a tissue section.

Preferably, the concentration of the agent for improving efficiency of small RNA extraction in the second mixture is from 20 to 60 vol%.

### [Advantageous Effects of Invention]

The agent for improving efficiency of small RNA extraction according to the present invention has excellent operability and stability, and can improve the efficiency of extraction of total RNA including miRNA by adding it to a nucleic acid extract containing a chaotropic salt in the already established Boom method. Therefore, by simply applying the agent for improving efficiency of small RNA extraction according to the present invention to a commercially available standard RNA extraction kit, miRNA can be extracted with high yield and high accuracy by an operation as safe and simple as the conventional one.

Moreover, according to the method of the present invention, the yield of miRNA is improved, which allows performance of quantitative analysis of low-expression miRNA with high accuracy.

### [Description of Embodiments]

Hereinafter, the present invention is described in detail. However, the present invention is not limited to the embodiments described in the present specification.

According to the first embodiment, the present invention is an agent for improving efficiency of small RNA extraction comprising the following components: (a) polyethylene glycol, and (b) tetraethylene glycol dimethyl ether.

"Small RNA" means RNA produced in a living organism with a length of about 10 nucleotides or more and less than 200 nucleotides. Examples include, but are not limited to, tRNA, rRNA, snRNA (small nuclear RNA), and miRNA (microRNA). A preferred small RNA according to the present embodiment is miRNA.

As the component (a) polyethylene glycol (hereinafter, also referred to as "PEG") according to the present embodiment, one with any average molecular weight can be used, and for example, PEG600, PEG1000, PEG1500, PEG1540, PEG2000, PEG3000, PEG4000, PEG6000, PEG8000, PEG10000, etc. can be used. Also, according to the present embodiment, one PEG selected therefrom may be used alone, or two or more thereof can be used in combination. The PEG according to the present embodiment is preferably PEG having an average molecular weight of 600 to 2000, particularly preferably PEG having an average molecular weight of 1000 to 2000, and most preferably PEG1000, PEG1540 or PEG2000.

In the agent for improving efficiency of small RNA extraction of the present embodiment, the component (a) PEG and the component (b) tetraethylene glycol dimethyl ether may be mixed at any ratio, e.g., at a ratio of 1:9, 2:8, 3:7, 4:6, 5:5, 6:4, 7:3, 8:2, or 9:1. Preferably, the component (a) and the component (b) may be mixed at a ratio of from 3:7 to 6:4, and more preferably, at a ratio of from 3:7 to 5:5.

As long as the agent for improving efficiency of small RNA extraction of the present embodiment contains the above components (a) and (b) at the above ratio, components other than the components (a) and (b) can be appropriately added within a range that does not undermine the effectiveness of the present embodiment. Specifically, the content of the component (a) PEG in the agent for improving efficiency of small RNA extraction of the present embodiment is preferably from 20 to 60% (v/v), particularly preferably from 45 to 55% (v/v), when the total volume of the agent for improving efficiency of small RNA extraction is 100%. In addition, the content of the component (b) tetraethylene glycol dimethyl ether in the agent for improving efficiency of small RNA extraction of the present embodiment is preferably from 25 to 70% (v/v), particularly preferably from 40 to 60% (v/v), when the total volume of the agent for improving efficiency of small RNA extraction is 100%.

In the present embodiment, the agent for improving efficiency of small RNA extraction preferably further contains a chaotropic salt as a component other than the above components (a) and (b). This allows stable storing of the agent for improving efficiency of small RNA extraction at room temperature for a longer period of time. Examples of the chaotropic salt according to the present embodiment include, but are not limited to, guanidine thiocyanate, guanidine hydrochloride, sodium thiocyanate, sodium iodide, potassium iodide, and urea. In the present embodiment, these chaotropic salts may be used alone or in combination of two or more. Preferably, the chaotropic salt according to the present embodiment is guanidine thiocyanate. The concentration of the chaotropic salt to be added to the agent for improving efficiency of small RNA extraction can be appropriately selected within the range of 20 to 30% (w/v), for example.

The agent for improving efficiency of small RNA extraction of the present embodiment may be appropriately blended with other components in order to further improve the effect and/or stability of small RNA extraction. Examples of the other components include 1-(2-aminoethyl)piperazine and diethylenetriamine, and these can be used alone or in combination of two or more. The concentration of the other components to be added to the agent for improving efficiency of small RNA extraction can be appropriately selected within the range of 0.01 to 5% (v/v), for example.

The method for producing the agent for improving efficiency of small RNA extraction of the present embodiment is not limited particularly, and it can be produced by mixing the above components.

The agent for improving efficiency of small RNA extraction of the present embodiment has a low viscosity and is stable at room temperature, and can be easily and accurately weighed. Specifically, the agent for improving efficiency of small RNA extraction of the present embodiment is preferably a liquid in the temperature range of 15 to 35°C, and has a viscosity that allows measuring by a micropipette. The "viscosity" according to the present embodiment can be determined using a rotational viscometer, for example. The viscosity of the agent for improving efficiency of small RNA extraction of the present embodiment is preferably 30 mPa·s or less, and particularly preferably 20 mPa·s or less.

The agent for improving efficiency of small RNA extraction of the present embodiment can be used in combination with a commercially available RNA extraction kit based on the Boom method, and allows extraction of small RNA with high yield and high accuracy by an operation as safe and simple as a conventional method.

Specifically, according to the second embodiment, the present invention is a method for extracting small RNA from a sample, the method comprising: (i) a step of providing a sample containing small RNA; (ii) a step of adding a solution containing a chaotropic salt to the sample; (iii) a step of adding the agent for improving efficiency of small RNA extraction as defined above to a first mixture obtained in the step (ii); (iv) a step of contacting a second mixture obtained in the step (iii) with a nucleic acid-binding carrier containing silica, whereby the small RNA binds to the nucleic acid-binding carrier; and (v) a step of eluting the small RNA which is bound to the nucleic acid-binding carrier.

In the method of the present embodiment, a sample containing small RNA is provided and is used. The "small RNA" according to the present embodiment is as defined in the first embodiment. The "sample" according to the present embodiment may be any sample containing small RNA, and may be, for example, a solution containing small RNA, a cell lysate, or the like. The cell lysate may be prepared from any cells, and can be prepared, e.g., from eukaryotic cells, prokaryotic cells, viruses, or the like. In addition, the sample according to the present embodiment may be a biological sample as described in detail in the third embodiment.

Subsequently, a solution containing a chaotropic salt is added to the sample containing small RNA to obtain a first mixture. The "chaotropic salt" according to the present embodiment is as defined in the first embodiment. The concentration of the chaotropic salt according to the present embodiment should be in accordance with the general conditions of the Boom method, and may be, e.g., in the range of 0.1 to 10 M, preferably 2.5 to 5 M in the second mixture after adding the agent for improving efficiency of small RNA extraction.

The solution containing a chaotropic salt according to the present embodiment may contain a reducing agent, a surfactant and/or a buffer in addition to the chaotropic salt. As the reducing agent, e.g., 2-mercaptoethanol dithiothreitol, or the like can be added, preferably at a concentration of from 25 to 150 mM. The surfactant may be ionic or nonionic, and for example, SDS, CHAPS, Tween (registered trademark) 20, Triton X-100 (registered trademark), NP-40 (registered trademark), or the like can be added, preferably at a concentration of from 0.1 to 10% (w/v). The buffer should stabilize the solution at a pH in the range of 6.0 to 9.0, and for example, Tris, HEPES, PIPES, MOPS or the like can be added, preferably at a concentration of from 1 to 10 mM.

The solution containing the chaotropic salt as described above is commercially available as a kit in combination with the nucleic acid-binding carrier as described below, and in the present embodiment, such commercial product can also be used. Examples of preferred commercial products include High Pure miRNA Isolation Kit (Roche Diagnostics), MagNA Pure Compact RNA Isolation Kit (Roche Diagnostics), RNeasy Mini Kit (Qiagen), miRNeasy Mini Kit (Qiagen), PureLink RNA Mini Kit (Thermo Fisher Scientific), PureLink miRNA Isolation Kit (Thermo Fisher Scientific), FastGene RNA Premium Kit (Nippon Genetics), Nucleo Spin miRNA (MACHEREY-NAGEL), and mirVana miRNA Isolation Kit (Thermo Fisher Scientific).

Subsequently, the agent for improving efficiency of small RNA extraction is added to the first mixture obtained as described above to obtain a second mixture. The "agent for improving efficiency of small RNA extraction" according to the present embodiment is as defined in the first embodiment. The agent for improving efficiency of small RNA extraction according to the present embodiment, can be preferably added, for example, at a concentration of from 20 to 60% (v/v), and particularly preferably added at a concentration of from 25 to 50% (v/v), in the second mixture after adding the agent for improving efficiency of small RNA extraction.

In the method of the present embodiment, the solution containing the chaotropic salt and the agent for improving efficiency of small RNA extraction can be added in interchangeable order, or can be added simultaneously. Specifically, a mixture obtained by adding the agent for improving efficiency of small RNA extraction to a sample containing small RNA may be prepared first, and then the solution containing a chaotropic salt may be added to the obtained mixture, or the solution containing a chaotropic salt and the agent for improving efficiency of small RNA extraction may be added simultaneously to the sample containing small RNA.

Subsequently, the second mixture obtained as described above is contacted with a nucleic acid-binding carrier containing silica so as to bind the RNA in the sample to the nucleic acid-binding carrier. As the nucleic acid-binding carrier according to the present embodiment, a silica-based carrier that is commonly used in the Boom method can be used, and, e.g., silica beads, glass beads, silica wool, glass fibers, or the like can be used. Also, the silica beads may consist only of silica, or may be magnetic silica beads, i.e., magnetic beads coated with silica. Such a nucleic acid-binding carrier is commercially available as a kit in combination with a solution containing a chaotropic salt as described above, and in the present embodiment, such a commercial product can also be used.

The mixture and the nucleic acid-binding carrier can be contacted, e.g., by mixing the mixture and the nucleic acid-binding carrier in a container, or by passing the mixture through a column filled with the nucleic acid-binding carrier. Then, the nucleic acid-binding carrier is separated and recovered from the mixture.

The recovered nucleic acid-binding carrier may optionally be washed by a washing solution that does not solubilize RNA but can solubilize non-specifically bound products (such as an aqueous solution containing 70% (v/v) or more ethanol), before performing the following elution of RNA.

Subsequently, the RNA bound to the nucleic acid-binding carrier is eluted. The RNA can be eluted, for example, by contacting an elution solution such as nuclease-free water or a buffer having a low salt concentration with a nucleic acid-binding carrier containing silica. The contact between the elution solution and the nucleic acid-binding carrier can be made in the same manner as the abovementioned contact between the mixture and the nucleic acid-binding carrier. Then, the nucleic acid-binding carrier is removed and the eluate is collected.

An automatic nucleic acid extraction device based on the Boom method is commercially available, and the above series of operations according to the method of the present embodiment can be performed using such a commercially available device and the kit provided therewith. Examples of the automatic nucleic acid extraction device and the kit provided therewith that can be used in the method of the present embodiment include MagNA Pure 96 Instrument and MagNA Pure 96 Cellular RNA Large Volume Kit (Roche Diagnostics), Maxwell RSC Instrument and Maxwell RSC simplyRNA Tissue Kit (Promega), Kingfisher Instrument and MagMAX mirVana Total RNA (Thermo Fisher Scientific), and QIAcube and RNeasy Mini QIAcube Kit (Qiagen).

In the method of the present embodiment, except that an agent for improving efficiency of small RNA extraction is used, the same solution and nucleic acid-binding carrier as those used in the conventionally known Boom method can be used, and the nucleic acid binding and elution conditions can be similar to those in the conventionally well-known Boom method. Therefore, according to the method of the present embodiment, small RNA can be extracted with high yield and high accuracy by an operation as safe and simple as the conventional method, without requiring a new examination of conditions.

Also, when the method of the present embodiment is applied to a biological sample, it is possible to easily extract miRNA with a low expression level, which was difficult by the conventionally known Boom method, without undermining the quantitative accuracy. Therefore, RNA isolated from a biological sample by the method of the present embodiment is useful for the quantitative analysis of miRNA.

Specifically, according to the third embodiment, the present invention is a method for analyzing microRNA from a biological sample, the method comprising: (i) a step of providing a biological sample containing microRNA; (ii) a step of adding a solution containing a chaotropic salt to the biological sample; (iii) a step of adding the above agent for improving efficiency of small RNA extraction to a first mixture obtained in the step (ii); (iv) a step of contacting a second mixture obtained in the step (iii) with a nucleic acid-binding carrier containing silica, whereby total RNA including the microRNA binds to the nucleic acid-binding carrier; (v) a step of eluting the total RNA which is bound to the nucleic acid-binding carrier; and (vi) a step of amplifying the microRNA in the total RNA obtained in the step (v) by quantitative RT-PCR.

In the method of the present embodiment, total RNA including microRNA is purified from a biological sample by the same procedure as in the method of the second embodiment. The "chaotropic salt", "agent for improving efficiency of small RNA extraction" and "nucleic acid-binding carrier" according to the present embodiment are as defined in the second embodiment.

"MicroRNA (miRNA)" is a single-stranded non-coding RNA having a length of from 21 to 25 bases that is present in living organisms. The miRNA is produced through the following multiple steps. First, a precursor pri-miRNA is transcribed from a miRNA gene. Next, the pri-miRNA is cleaved by Drosha in the nucleus to yield a pre-miRNA. Next, the pre-miRNA is cleaved by Dicer in the cytoplasm to yield a mature double-stranded miRNA, of which one of the RNA strands is decomposed and removed to generate a single-stranded miRNA. The miRNA according to the present embodiment refers to this final miRNA product, and does not include the intermediate products.

The "biological sample" for the method of the present embodiment may be any one isolated from any living organism. For example, it may be a tissue section, a cell, a body fluid or the like isolated from an animal individual such as a mouse, rat, rabbit, dog, non-human primate, or human, but is not particularly limited thereto. Examples of tissues from which the tissue sections or cells are derived include the brain, cardiac muscle, skeletal muscle, lung, trachea, pharynx, salivary gland, esophagus, stomach, small intestine, large intestine, pancreas, liver, gallbladder, blood vessel, kidney, bladder, ureter, testis, prostate, ovary, uterus, bone marrow, lymph node, spleen, thymus, pituitary gland, thyroid, parathyroid gland, adrenal gland, skeletal muscle, abdominal cavity, skin, and mammary gland. Examples of body fluids include blood, plasma, serum, saliva, urine, gastric juice, pancreatic juice, bile, sweat, tears, breast milk, nasal discharge, and intestinal juice. The biological sample according to the present embodiment is preferably a body fluid or a tissue section, and is particularly preferably a human-derived body fluid or a tissue section.

The biological sample according to the present embodiment can be obtained from an animal individual by a well-known method for one skilled in the art. In addition, when a tissue section is used as the biological sample, the tissue section may be prepared from tissue acutely isolated from an animal individual, or may be prepared from a formalin-fixed paraffin-embedded tissue (FFPET).

Next, the miRNA in the obtained total RNA is amplified by quantitative RT-PCR (hereinafter, referred to as "RT-qPCR"). The methods for amplifying miRNA by RT-qPCR have already been established, and a method well known in the art can be adopted. For example, the target miRNA can be amplified by performing a reverse transcription reaction using a primer specific to miRNA, and using the resulting cDNA as a template to perform real-time qPCR using a primer specific to the target miRNA. In addition, the methods for quantitative analysis are also well established, and the target miRNA can be quantified by, for example, the ΔΔCt method, the calibration curve method, or the like, based on the Cp value (also referred to as Ct value) calculated from the real-time qPCR results.

Kits for amplifying miRNA by RT-qPCR are commercially available, and such commercial products can be used in the present embodiment. Examples of preferred commercial products include miRCURY LNA miRNA PCR Assays (Qiagen), MystiCp micro RNA Quantitative PCR (Sigma-Aldrich), Mir-X miRNA qRT-PCR TB Green Kit (Takara Bio), TaqMan MicroRNA Assays and TaqMan Advanced miRNA Assays (Thermo Fisher Scientific).

The method of the present embodiment is useful since using the agent for improving efficiency of small RNA extraction allows extraction of miRNA even with a low expression level without undermining quantitative accuracy and to quantify miRNA with high accuracy.

### [Examples]

Hereinafter, the present invention will be further described with reference to the Examples. However, the present invention is not in any way limited thereto.

### 1. Search for compound that improve miRNA extraction efficiency

A standard sample containing miRNA was prepared according to the following procedure. A solution (100 nM) of chemically synthesized miR-21 (5'-UAGCUUAUCAGACUGAUGUUGA-3': SEQ ID NO: 1) (Hokkaido System Science) was prepared using a TE buffer (pH 8.0) containing 10 ng/µL of salmon sperm DNA. To the resulting miR-21 solution (2.5 µL), total RNA (100 ng) extracted from K562 cells (obtained from JCRB Cell Bank, National Institutes of Biomedical Innovation, Health and Nutrition) was added, and then 150 µL of a solution containing the mixture was prepared with distilled water as a standard sample.

The miRNA from the standard sample was extracted by using a High Pure miRNA Isolation Kit (Roche Diagnostics) and according to the " 1-Column Protocol" described in the instruction manual provided with the kit. Here, in the step of adding the Binding Enhancer (hereinafter referred to as "BE") provided with the kit, the following candidate compounds were added instead of BE (200 µL): ethanol (Wako Pure Chemical), 2-propanol (Kanto Chemical), PEG600 (Kanto Chemical), PEG1000 (Kanto Chemical), PEG1540 (Kanto Chemical), PEG2000 (Kanto Chemical), or tetraethylene glycol dimethyl ether (hereinafter referred to as "TEGDME") (Tokyo Chemical Industry) (all 200 µL). The elution of RNA was performed with 100 µL of Elution Buffer.

Next, cDNA was synthesized using Transcriptor First Strand cDNA Synthesis Kit (Roche Diagnostics). As the primer, a stem loop RT primer (5'-GTCAGAGGAGGTGCAGGGTCCGAGGTATTCGCACCTCCTCTGACTCAACA-3': SEQ ID NO: 2) was used. The composition of the reaction solution was in accordance with the description of the instruction manual provided with the kit (1/2 scale). The reverse transcription reaction was performed using an Applied Biosystems (trademark) 2720 thermal cycler under the following conditions.

### [Reaction conditions]

- Step 1 (1 cycle): 16°C for 30 minutes
- Steps 2 to 4 (60 cycles): 30°C for 30 seconds, 42°C for 30 seconds, 50°C for 1 second
- Step 5 (1 cycle): 85°C for 5 minutes

The resulting reaction solution was diluted 5-fold with TE buffer to prepare a cDNA solution. Subsequently, real-time PCR was performed on miR-21 under the following conditions. For the reaction and analysis, LightCycler (registered trademark) 96 System (Roche Diagnostics) was used. The reaction and analysis were performed in quintuplicate, and Cp values (mean ± SD) were calculated.

### [Reaction solution composition (10 µL)]

- cDNA solution: 2.5 µL
- 2 x FastStart Essential DNA Probes Master (Roche Diagnostics): 5 µL
- 10 µM forward primer (5'-GCCTGCTAGCTTATCAGACTGATG -3': SEQ ID NO: 3): 0.2 µL
- 10 µM reverse primer (5'-GTGCAGGGTCCGAGGT-3': SEQ ID NO: 4): 0.2 µL
- 10 µM Universal Probe Library Probe #82 (Roche Diagnostics): 0.2 µL
- Distilled water: remaining amount

### [Reaction conditions]

- Step 1 (1 cycle): 95°C for 10 minutes
- Steps 2 to 3 (45 cycles): 95°C for 10 seconds, 60°C for 30 seconds

The results are shown in Table 1. When PEG1000, PEG1540 or PEG2000 was used instead of BE, the Cp value was significantly reduced compared to when BE was used. On the other hand, when PEG600 or TEGDME was used, the Cp value was almost the same as when BE was used, and when ethanol or 2-propanol was used, the Cp value was significantly increased compared to when BE was used. These results show that high-molecular-weight PEG can remarkably improve the miRNA extraction efficiency.

Table 1. Cp value for miR-21 (standard sample)

**[Table 1]**

| Compound | Cp value |
|---|---|
| Ethanol | 16.65 ± 0.27 |
| 2-propanol | 16.40 ± 0.42 |
| PEG600 | 14.00 ± 0.78 |
| PEG 1000 | 13.01 ± 0.43 |
| PEG 1540 | 12.70 ± 0.36 |
| PEG2000 | 12.37 ± 0.35 |
| TEGDME | 14.48 ± 0.20 |
| BE (control) | 14.60 ± 0.36 |

### 2. Examination of composition improving miRNA extraction efficiency (1)

High-molecular-weight PEGs can remarkably improve the miRNA extraction efficiency; however, they are solid at room temperature and are extremely viscous even if heated and melted, and are therefore difficult to accurately measure them with a pipette. Thus, we examined whether the above problem could be solved by mixing high-molecular-weight PEGs with TEGDME Compositions in which PEG1540 and TEGDME were mixed at various ratios were prepared and incubated at 60°C. Then, the viscosity was measured using a viscometer (VISCO-895, ATAGO) in an incubator at 50°C. The measurement was performed in quintuplicate and the mean ±SD was used as the measured value. In addition, the Cp value when adding each composition was calculated by the same procedure as in the above 1 (addition amount: 200 µL or 300 µL, duplicate measurement).

The results are shown in Table 2. Mixing PEG1540 with TEGDME resulted in a considerable reduction in viscosity and a Cp value significantly lower than BE. These results show that the compositions of PEG1540:TEGDME=3:7 to 6:4 can remarkably improve the yield of miRNA and has excellent operability.

Table 2. Viscosity and Cp value of PEG1540/TEGDME mixture

**[Table 2]**

| Composition (PEG1540:TEGDME) | Viscosity (mPa·s) | Cp value (200 µL) | Cp value (300 µL) |
|---|---|---|---|
| 10:0 | 132.8 ± 2.1 | 12.52 | 12.41 |
| 7:3 | 39.5 ± 1.0 | 12.80 | 12.33 |
| 6:4 | 27.6 ± 0.1 | 13.05 | 12.45 |
| 5:5 | 21.8 ± 0.3 | 13.27 | 12.47 |
| 4:6 | 14.9 ± 0.3 | 13.32 | 12.53 |
| 3:7 | 10.2 ± 0.2 | 13.53 | 12.40 |
| 0:10 | 1.7 ± 0.2 | 14.93 | 12.87 |
| BE (control) | - | 15.00 | - |

Furthermore, PEG600, PEG1000, or PEG2000 was used instead of PEG1540 to prepare a PEG/TEGDME mixture (mixing ratio 1:1). The viscosity was measured and the Cp values were calculated (addition amount: 200 µL or 300 µL, quintuplicate measurement) in the same manner as above.

The results are shown in Table 3. It was confirmed that any of the high-molecular-weight PEG/TEGDME mixtures had a viscosity (about 30 mPa·s or less) that allows accurate measuring by a pipette, and in particular, an addition amount of 300 µL can remarkably improve the yield of miRNA. In addition, a similarly low Cp value was obtained for the mixtures using any of the high-molecular-weight PEGs, within the range of high-molecular-weight PEG:TEGDME=3:7 to 6:4 (data not shown).

Table 3. Viscosity and Cp value of high-molecular-weight PEG/TEGDME

**[Table 3]**

| Composition | Viscosity (mPa·s) | Cp value (200 µL) | Cp value (300 µL) |
|---|---|---|---|
| PEG600/TEGDME | 14.3 ± 0.1 | 14.24 ± 0.19 | 12.47 ± 0.03 |
| PEG1000/TEGDME | 18.0 ± 1.2 | 13.65 ± 0.12 | 12.32 ± 0.08 |
| PEG2000/TEGDME | 30.7 ± 0.3 | 12.96 ± 0.10 | 12.05 ± 0.07 |
| BE (control) | - | 14.24 | - |

### 3. Examination of composition improving miRNA extraction efficiency (2)

PEG1000/TEGDME (mixing ratio 1:1), PEG1540/TEGDME (mixing ratio 1:1) and PEG2000/TEGDME (mixing ratio 1:1) were solidified about 1 hour after preparation. Thus, it is necessary to remelt them by heating at the time of use. However, almost no change was observed when measuring the Cp values after repeating solidification/remelting up to 10 times (data not shown). Therefore, it was confirmed that all of the high-molecular-weight PEG/TEGDME mixtures had sufficient stability; however, compositions that are not solidified at room temperature and also have high miRNA extraction efficiency were further investigated.

Based on PEG1540/TEGDME (mixing ratio 1:1) (composition 1), the compositions 2 to 6 were prepared, which contain the additional components: guanidine thiocyanate (hereinafter referred to as "GuSCN") (Wako Pure Chemical), ethanol (Wako Pure Chemical), 1-(2-aminoethyl)piperazine (hereinafter referred to as "piperazine") (Sigma-Aldrich) and/or diethylenetriamine (hereinafter referred to as "DETA") (Sigma-Aldrich).

Table 4. Preparation of PEG1540/TEGDME based compositions

**[Table 4]**

| Composition | GuSCN (25% (v/v)) | Ethanol (5% (v/v)) | Piperazine (1 % (v/v)) | DETA (0.5% (v/v)) |
|---|---|---|---|---|
| 1 | - | - | - | - |
| 2 | + | - | - | - |
| 3 | - | + | + | - |
| 4 | - | + | - | + |
| 5 | + | + | + | - |
| 6 | + | + | - | + |

The Cp values when the above compositions were added were calculated by the same procedure as in the above 1 (addition amount: 200 µL or 300 µL, quintuplicate measurement).

Also, the above compositions (1 mL) that had been left for 1 hour at 25°C after preparation were visually observed to confirm whether there was solidification.

### [Criteria]

No aggregation (no solidification): -
Some aggregates (partially solidified): ±
Aggregation (complete solidification): +

The results are shown in Table 5. The composition to which GuSCN was added was not solidified at room temperature. The same results were also obtained with the composition that had been left for one week or more at room temperature (data not shown). In addition, lower Cp values were obtained by adding ethanol, piperazine and/or DETA in combination with GuSCN. These results show that the addition of GuSCN, ethanol, piperazine and/or DETA can further improve the efficiency of RNA extraction and the stability of the high-molecular-weight PEG/TEGDME mixture.

Table 5. Cp values and solidification of PEG1540/TEGDME based compositions

**[Table 5]**

| Composition | Cp value (200 µL) | Cp value (300 µL) | Solidification |
|---|---|---|---|
| 1 | 12.79 ± 0.20 | 12.07 ± 0.14 | + |
| 2 | 15.18 ± 0.15 | 12.24 ± 0.25 | - |
| 3 | 12.37 ± 0.11 | 11.82 ± 0.37 | ± |
| 4 | 12.76 ± 0.20 | 11.93 ± 0.12 | + |
| 5 | 13.21 ± 0.10 | 12.72 ± 0.15 | - |
| 6 | 13.97 ± 0.21 | 12.12 ± 0.10 | - |
| BE (control) | 14.89 | - | - |

### 4. RNA extraction from FFPET sample using high-molecular-weight PEG/TEGDME mixture

Instead of a standard sample, FFPET samples obtained from a cancer patient were used to test to what extent a high-molecular-weight PEG/TEGDME mixture could improve the miRNA extraction efficiency from a biological sample.

An FFPET section (thickness: 5 µm) prepared from the large intestine taken from a colorectal cancer patient was recovered from a glass slide into a microtube, to which 800 µL of xylene was added, and the mixture was incubated at room temperature for 5 minutes. Then, 400 µL of ethanol was added and mixed. The mixture was centrifuged at 13000 rpm for 2 minutes and the supernatant was removed. 1000 µL of ethanol was added and mixed again. The mixture was centrifuged at 13000 rpm for 2 minutes and the supernatant was removed. Then, 180 µL of Paraffin Tissue Lysis Buffer and 70 µL of proteinase K solution provided with the High Pure miRNA Isolation Kit (Roche Diagnostics) were added, and the mixture was incubated at 56°C for 30 minutes, and then incubated at 90°C for 30 minutes.

The high-molecular-weight PEG/TEGDME mixture was added to extract miRNA, RT-qPCR was performed on miR-21 and the Cp values were calculated (addition amount: 200 µL or 300 µL, quintuplicate measurement) by the same procedure as in the above 1, except that the resulting tissue lysate (150 µL) was used instead of the standard sample,

The results are shown in Table 6. The Cp values were significantly reduced by using any of the high-molecular-weight PEG/TEGDME mixtures, compared to the case using BE. These results showed that a high-molecular-weight PEG/TEGDME mixture can remarkably improve the miRNA extraction efficiency from an FFPET sample.

Table 6. Cp values of miR-21 contained in RNA extracted from FFPET sample

**[Table 6]**

| Composition (mixing ratio 1:1) | Cp value (200 µL) | Cp value (300 µL) |
|---|---|---|
| PEG600/TEGDME | 23.41 ± 0.91 | 20.69 ± 0.74 |
| PEG1000/TEGDME | 22.63 ± 0.89 | 20.42 ± 0.70 |
| PEG1540/TEGDME | 22.31 ± 0.95 | 20.38 ± 0.90 |
| PEG2000/TEGDME | 22.02 ± 0.96 | 20.47 ± 0.83 |
| BE (control) | 24.17 ± 0.93 | - |

These results demonstrated that compositions containing high-molecular-weight PEG and TEGDME can remarkably improve the miRNA extraction efficiency, and are useful for more accurately quantifying miRNA.

### 5. RNA extraction from body fluid sample using high-molecular-weight PEG/TEGDME mixture

We used a serum sample from a healthy subject instead of a standard sample, and tested to what extent a high-molecular-weight PEG/TEGDME mixture could improve the miRNA extraction efficiency from a body fluid sample.

Whole blood samples were collected from 5 healthy subjects and centrifuged at 3000 rpm for 10 minutes to recover the serum. The obtained serum samples were immediately frozen and stored at -80°C. 100 µL of Proteinase K, recombinant, PCR Grade (Roche Diagnostics) prepared at 20 mg/mL was added to 1 mL of the serum, and the mixture was incubated at 72°C for 10 minutes. Then, the mixture was centrifuged at 13000 rpm for 5 minutes and the supernatant was recovered.

The PEG1540/TEGDME mixture (mixing ratio 1:1) was added to extract miRNA, RT-qPCR was performed on miR-21 and the Cp values were calculated (addition amount: 200 µL or 300 µL, quintuplicate measurement) by the same procedure as in the above 1, except that the obtained supernatant (150 µL) was used instead of the standard sample.

The results are shown in Table 7. Generally, a Cp value of around 35 is regarded as the limit of quantification in RT-qPCR. When the Cp value exceeds 35, the variation of the Cp value increases and the quantification accuracy is considered to be unreliable. Here, when BE was used, the Cp value significantly exceeded 35 and the standard deviation also exceeded 1, and the results were quantitatively unreliable. In contrast, when the PEG1540/TEGDME mixture was used, in particular when the addition amount was 300 µL, the Cp value was greatly reduced, and could obtain reliable quantification analysis results with a sufficiently small standard deviation.

miR-21 is a tumor marker, and has been known to be contained in body fluid samples from a healthy subject in only extremely small amounts. As the above results show, it was demonstrated that, even for such miRNAs with a low expression level, using a high-molecular-weight PEG/TEGDME mixture can be remarkably improved the miRNA extraction efficiency and can enable reliable quantitative analysis by RT-qPCR.

Table 7. Cp values of miR-21 contained in RNA extracted from serum sample

**[Table 7]**

| Composition (mixing ratio 1:1) | Cp value (200 µL) | Cp value (300 µL) |
|---|---|---|
| PEG1540/TEGDME | 35.19 ± 0.62 | 32.72 ± 0.43 |
| BE (control) | 36.71 ± 1.10 | - |

## Claims

1. An agent for improving efficiency of small RNA extraction comprising the following components:
(a) polyethylene glycol, and
(b) tetraethylene glycol dimethyl ether.

2. The agent for improving efficiency of small RNA extraction according to claim 1, wherein the polyethylene glycol has an average molecular weight of from 600 to 2000.

3. The agent for improving efficiency of small RNA extraction according to claim 1 or 2, wherein a ratio of the component (a) to the component (b) is from 3:7 to 6:4.

4. The agent for improving efficiency of small RNA extraction according to any one of claims 1 to 3, wherein a content of the component (a) is from 20 to 60 vol%, and a content of the component (b) is from 25 to 70 vol%, based on the agent for improving efficiency of small RNA extraction.

5. The agent for improving efficiency of small RNA extraction according to any one of claims 1 to 4, further comprising a chaotropic salt.

6. The agent for improving efficiency of small RNA extraction according to claim 5, wherein the chaotropic salt is guanidine thiocyanate.

7. The agent for improving efficiency of small RNA extraction according to any one of claims 1 to 6, wherein the agent is a liquid in a temperature range of 15 to 35°C, and has a viscosity that allows measuring by a micropipette.

8. A method for extracting small RNA from a sample, the method comprising:
(i) a step of providing a sample comprising small RNA;
(ii) a step of adding a solution comprising a chaotropic salt to the sample;
(iii) a step of adding the agent for improving efficiency of small RNA extraction according to any one of claims 1 to 7 to a first mixture obtained in the step (ii);
(iv) a step of contacting a second mixture obtained in the step (iii) with a nucleic acid-binding carrier containing silica, whereby the small RNA binds to the nucleic acid-binding carrier; and
(v) a step of eluting the small RNA which is bound to the nucleic acid-binding carrier.

9. A method for analyzing microRNA in a biological sample, the method comprising:
(i) a step of providing a biological sample comprising microRNA;
(ii) a step of adding a solution comprising a chaotropic salt to the biological sample;
(iii) a step of adding the agent for improving efficiency of small RNA extraction according to any one of claims 1 to 7 to a first mixture obtained in the step (ii);
(iv) a step of contacting a second mixture obtained in the step (iii) with a nucleic acid-binding carrier containing silica, whereby total RNA including the microRNA binds to the nucleic acid-binding carrier;
(v) a step of eluting the total RNA which is bound to the nucleic acid-binding carrier; and
(vi) a step of amplifying the microRNA in the total RNA obtained in the step (v) by quantitative RT-PCR.

10. The method according to claim 9, wherein the biological sample is a body fluid or a tissue section.

11. The method according to any one of claims 8 to 10, wherein a concentration of the agent for improving efficiency of small RNA extraction in the second mixture is from 20 to 60 vol%.
